# EUROPÄISCHE PATENTSCHRIFT

(11) **EP 1 288 194 B1**
(45) Veröffentlichungstag und Bekanntmachung des Hinweises auf die Patenterteilung: **03.10.2007**
(21) Anmeldenummer: 02016059.4
(22) Anmeldetag: 19.07.2002
(51) Int. Cl.: C07C 249/14, C07C 251/44

(54) **Aufarbeitung der Ammoximationsprodukte von Ketonen durch Flüssig-Flüssig-Extraktion in einem ternären Lösemittelsystem**
Working-up of the ammoximation products of ketones using liquid-liquid extraction
Traitement des produits d'ammoxymation des cétones par extraction liquide-liquide dans un système de solvant ternaire

(30) Priorität: 31.08.2001 DE 10142621
(43) Veröffentlichungstag der Anmeldung: 05.03.2003
(73) Patentinhaber: Degussa GmbH, 40474 Düsseldorf (DE)
(72) Erfinder: Schiffer, Thomas, Dr., 45721 Haltern (DE); Esser, Peter Ernst, Dr., 85609 Aschheim (DE); Krissmann, Jörg, Dr., 45772 Marl (DE); Roos, Martin, Dr., 45721 Haltern (DE); Stevermüer, Günter, 45770 Marl (DE); Thiele, Georg-Friedrich, Dr., 63450 Hanau (DE)

(56) Entgegenhaltungen:
- EP-A- 0 208 311
- EP-A- 0 267 362
- EP-A- 0 496 385

## Beschreibung

Die Erfindung betrifft die Aufarbeitung der Ammoximationsprodukte von Ketonen wie Alkanonen oder Cycloalkanonen mit vorzugsweise 8-20 Kohlenstoffatomen durch Flüssig-Flüssig-Extraktion in einem ternären Lösemittelsystem.

In zahlreichen Patentanmeldungen und Patentschriften wird die Ammoximation von Alkanonen und/oder Cycloalkanonen mit Wasserstoffperoxid und Ammoniak an einem heterogenen Katalysatorsystem, welches mindestens eine Komponente enthält, die aus den Elementen Titan, Silizium und Sauerstoff aufgebaut ist, beschrieben. Als Beispiele seien hier die EP 0 299 430 (Montedipe), EP 0 564 040 (Enichem) und US 5 637 715 (Degussa) genannt.

In der Regel wird als Katalysator ein mikro- oder mesoporöser Titanzeolith verwendet, wobei sich der Titansilikalit TS1 besonders gut für die Ammoximation eignet. Bei großen und sterisch anspruchsvollen Alkanonen oder Cycloalkanonen ist es außerdem vorteilhaft, das Katalysatorsystem durch weitere Komponenten zu ergänzen. So werden in DE 195 21 011 (Enichem) amorphe Silikate, in DE 100 47 435 (Degussa-Hüls) saure Feststoffe und in DE 101 03 581 (Degussa-Hüls) Ammoniumionen als Cokatalysatoren beansprucht.

Wie die Patentanmeldungen DE 100 47 435 und DE 101 03 581 zeigen, verläuft die Reaktion bei großen und sterisch anspruchsvollen (Cyclo-)Alkanonen, wie zum Beispiel Cyclododecanon, besonders schnell und selektiv in polaren, mit Wasser ganz oder teilweise mischbaren organischen Lösemitteln, insbesondere in kurzkettigen Alkoholen mit 1 bis 6 Kohlenstoffatomen.

Die Ammoximation erfolgt in zwei Teilschritten umfassend die Hydroxylaminbildung (1) und die Oximierung (2), dargestellt am Beispiel der Ammoximation von Cyclododecanon (CDON). Wasser wird dabei zum einen durch eine wässrige Wasserstoffperoxidlösung eingetragen, zum anderen bildet sich Wasser in stöchiometrischen Mengen bei den beiden Teilschritten als Reaktionsprodukt.

Zusätzlich entsteht Wasser auch bei der unproduktiven Zersetzung von Wasserstoffperoxid und Hydroxylamin, formal dargestellt in den Nebenreaktionen (3) und (4).

(1) NH₃+H₂O₂ → H₂O +NH₂OH

(2) NH₂OH+ CDON → CDON-Oxim+H₂O

(3) 2NH₂OH +H₂O₂ → 4H₂O + N₂

(4) 2H₂O₂ → 2H₂O + O₂

Während der Umsetzung steigt folglich der Gehalt an Wasser in der Reaktionsmischung an. Sollen große Alkanone oder Cycloalkanone, wie zum Beispiel Cyclododecanon, ammoximiert werden, so fällt insbesondere die Löslichkeit des entsprechenden Oxims in der Reaktionsmischung mit steigendem Wassergehalt stark ab.
Ziel der Reaktionsführung insbesondere bei großen Cycloalkanonen ist daher, die Menge an Wasser während der Reaktion möglichst zu begrenzen. Dies gelingt gemäß DE 100 47 435 und DE 101 03 581 zum Beispiel dadurch, dass vorteilhaft Ammoniak als trockenes Gas und Wasserstoffperoxid als möglichst konzentrierte Lösung (üblicherweise ≥ 30 Gew.%) verwendet werden. Auch ist es vorteilhaft, wenn der als Lösemittel eingesetzte Alkohol zu Beginn der Reaktion nicht mehr Wasser enthält, als nach einer Destillation im Azeotrop enthalten ist.
Soll der Alkohol mehrfach im Prozess verwendet werden, muss die bei der Reaktion eingetragene Menge an Wasser bei der Aufarbeitung wieder abgetrennt werden.

In den meisten Patentanmeldungen steht die Synthese des Katalysatorsystems, seine Aktivierung und die Ammoximationsreaktion selbst im Mittelpunkt der Untersuchungen. Zur Aufarbeitung findet sich in den oben erwähnten Schriften der allgemeine Hinweis, dass der meist pulverförmige Katalysator, in der Regel ein Titansilikalit, über ein Filter oder eine Drucknutsche abgetrennt wird. Anschließend werden Umsätze und Selektivitäten durch GC-Analyse und der Peroxidverbrauch durch Redoxtitration direkt aus der Reaktionslösung bestimmt.

ARCO-Chemical Technology beschreibt in EP 0 690 045 und EP 0 735 017 einen mehrstufigen Syntheseprozess. Hierbei wird zunächst Wasserstoffperoxid durch die Reaktion von Isopropanol mit Sauerstoff gebildet. Nach Abtrennen und Hydrieren des ebenfalls gebildeten Acetons bewirkt Wasserstoffperoxid mit Ammoniak die Ammoximation von Cyclohexanon. Daran schließt sich die Beckmann-Umlagerung zu Caprolactam an.

Für den Prozessschritt der Ammoximation von Cyclohexanon wird allgemein jedes geeignete Aufarbeitungsverfahren beansprucht. Als Möglichkeiten werden die Destillation und die Extraktion genannt, ohne diese beiden Verfahren mit experimentellen Daten oder Beispielen zu konkretisieren.

Eine vollständige destillative Trennung von Lösemittel, Edukt und Produkt, wie in US 5 451 701 (entspricht EP 0 690 045 (Arco Chemical Technologies)) beschrieben, mag bei Cyclohexanon-Oxim noch möglich sein. Nach Destillation des Lösemittels und Wassers lassen sich Cyclohexanon (Sdp. 155 °C / 1013 mbar) und Cyclohexanon-Oxim (Sdp. 206 - 210 °C /1013 mbar) voneinander trennen, vorteilhaft erfolgt diese Destillation im Vakuum.

Für die Ammoximation makrocyclischer Ketone wie zum Beispiel Cyclododecanon ist ein rein destillatives Verfahren jedoch nicht mehr geeignet. Die destillative Trennung von Keton und Oxim wird mit zunehmender Ringgröße schwieriger, zudem bewirken die hohen Destillationstemperaturen auch im Hochvakuum erhebliche Anteile an Zersetzung. Cyclododecanon-Oxim lässt sich nicht mehr ohne Zersetzung destillieren.

Montedipe beschreibt in EP 0 208 311, Beispiel 1, die Umsetzung und Aufarbeitung der Ammoximation von Cyclohexanon ohne Alkohol als Lösemittel in einem Dreiphasengemisch (organisch-wässrig-fest) bestehend aus Cyclohexanon als organischer Phase, 32gew.-%igem wässrigem Ammoniak und 32gew.-%igem Wasserstoffperoxid als wässrige Phase, sowie pulverförmigem Titansilikalit als festem Katalysator. Nachteilig bei dem Verfahren ist, dass beim Abkühlen der Reaktionsmischung die organische Phase, bestehend aus Cyclohexanon-Oxim und nicht umgesetztem Cyclohexanon auskristallisiert und dabei den Katalysator einschließt. Zur Aufarbeitung und Abtrennung des Katalysators muss die organische Phase wieder in Toluol gelöst und die wässrige Phase mehrfach mit Toluol extrahiert werden. Für diskontinuierliche Ansätze im Labor mag dieses Verfahren geeignet sein, doch lässt sich dieses Verfahren nicht oder nur mit hohem apparativen Aufwand in einen kontinuierlichen technischen Prozess überführen.

Das Aufarbeitungsverfahren der Extraktion wird auch von Montedipe in US 4 794 198 (entspricht EP 0 267 362) am Rande erwähnt, bei der mit Wasser mischbare Lösemittel, wie zum Beispiel wässriges tert.-Butanol als Lösemittel der Ammoximation verwendet werden. Ein geeignetes organisches Solvens wird der Reaktionsmischung am Ende der Reaktion zugesetzt und anschließend das Oxim mit dem Solvens vom wässrigen Lösemittel abgetrennt.

In den diskontinuierlichen Versuchen wurde nach Abkühlen der Reaktionsmischung zur erhaltenen Suspension Diethylether (Beispiel 3, 20) zugesetzt, der Katalysator anschließend abfiltriert und die organische Phase abdekantiert. Bei den kontinuierlichen Versuchen mit Katalysator-Suspension (Beispiel 32) und beim Rieselbett (Beispiel 33) werden keine Angaben zur Aufarbeitung gemacht.

In der europäischen Patentanmeldung EP 0 267 362 (Montedipe) wird neben der Umsetzung von Cyclohexanon auch die Umsetzung einiger anderer Carbonylverbindungen wie zum Beispiel von Cyclododecanon beansprucht. Ein konkretes Beispiel mit Cyclododecanon wird jedoch nicht angegeben.

Ein kontinuierliches Verfahren zur Ammoximation beschreibt Enichem in EP 0 496 385. Nach der Isolierung des Katalysators wird zunächst ein ammoniakhaltiges Azeotrop der Lösemittel tert.-Butanol und Wasser in einer ersten Kolonne (mit C1 bezeichnet) von der Reaktionsmischung abgetrennt. Im Sumpf dieser Kolonne sammelt sich die verbliebene Reaktionsmischung, bestehend aus Cyclohexanon-Oxim (Smp. 95 °C), weiteren Nebenkomponenten und restlichem Wasser. Das Oxim wird anschließend in einem Extraktor mit Toluol aus der Reaktionmischung heraus gewaschen.

In EP 0 496 385, Anspruch 8, werden explizit die Carbonylverbindungen Aceton, Cyclohexanon, Methylethylketon, Acetophenon, Cyclododecanon und der Önanthaldehyd genannt. In den aufgeführten Beispielen 1 - 5 wird das Verfahren allerdings nur auf Cyclohexanon angewendet.

Die Schwierigkeit bei diesem Aufarbeitungsprozess liegt bei großen Keton-Oximen, wie zum Beispiel Cyclododecanon-Oxim, darin, dass einerseits das Oxim in Wasser nur noch schlecht löslich ist, andererseits der Schmelzpunkt des Oxims oberhalb des Siedepunktes von Wasser liegt. Das in EP 0 496 385 beschriebene Aufarbeitungsverfahren ist somit für größere Oxime als Cyclohexanon-Oxim nicht oder nur noch eingeschränkt geeignet. Beim Nacharbeiten des Versuches kristallisierte jeweils Cyclododecanon-Oxim im Abtriebsteil der Kolonne aus.

Es bestand daher die Aufgabe, ein kontinuierliches Aufarbeitungsverfahren für die Ammoximation von Ketonen, insbesondere von großen Cycloalkanonen, mit 8-20 Kohlenstoffatomen zu entwickeln, bei dem
der Katalysator nach der Reaktion abgetrennt wird,
das Oxim aus der Reaktionsmischung isoliert wird,
das Reaktionswasser vom Lösemittel abgetrennt wird und
der verbleibende Alkohol in den Prozess zurückgeführt wird,
ohne dass während der genannten Schritte des Aufarbeitungsprozesses das Produkt als Feststoff ausfällt.

Überraschend wurde nun gefunden, dass sich der geforderte Aufarbeitungsprozess dadurch realisieren lässt, dass man für die Aufarbeitung ein ternäres Lösemittelsystem verwendet, das erstens aus einem oder mehreren vollständig oder gut mit Wasser mischbaren organischen Flüssigkeiten wie insbesondere Alkoholen, zweitens aus Wasser und drittens aus einem mit Wasser und Alkohol nur partiell mischbaren organischen Lösemittel besteht, dessen Siedepunkt oberhalb der Siedepunkte von Alkohol und Wasser liegt, und dass zur Extraktion die Mischungslücke des ternären Lösemittelsystems durch Variation des Wassergehaltes ausgenutzt wird und dabei die Grenzdestillationslinie überschritten wird.

Es können gegebenenfalls auch mehrere Extraktionsstufen verwendet werden.

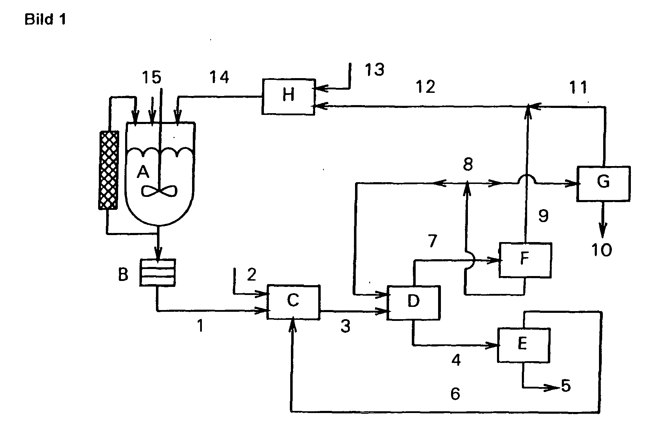

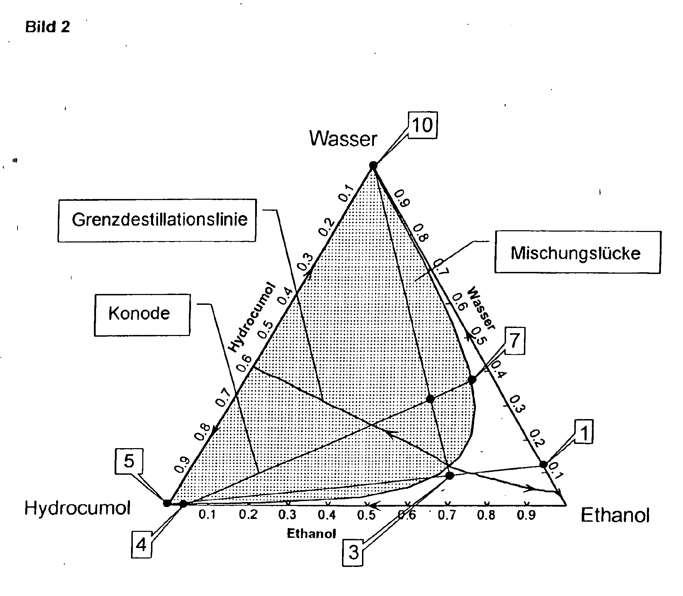

Das erfindungsgemäße Verfahren ist in Bild 1 skizziert. Es beruht im Wesentlichen auf der Mischungslücke, die sich zwischen einer alkoholisch-wässrigen Phase und einem unpolaren Kohlenwasserstoff ergibt. Bild 2 zeigt die Mischungslücke am Beispiel des ternären Systems Ethanol, Wasser und Hexahydrocumol.
Erfindungsgemäß wird zunächst der Reaktionsaustrag vom Katalysator befreit.

Wird Titansilikalit-Katalysator im Reaktor A in Pulverform verwendet, wird dieser zunächst in einem Separationsschritt B abgetrennt. Dafür eignen sich technisch beispielsweise Filterkerzen, eine Drucknutsche oder eine Filterzentrifuge sehr gut. Wird, wie in Bild 1 skizziert, ein Umlaufreaktor (A) mit Katalysator-Formkörpern als Festbett eingesetzt, entfällt die Katalysatorabtrennung. Als Separationsschritt B wird dann zur Sicherheit ein Reinigungsfilter verwendet, um feste Verunreinigungen, Schwebeteilchen oder Abriebspuren des Formkörpers aus der Reaktionsmischung zurückzuhalten.

Der vom Katalysator befreite Reaktoraustrag (1) besteht aus dem jeweiligen Keton-Oxim, vollständig gelöst in einem mit Wasser gut oder vollständig mischbaren Alkohol mit 1 bis 6 Kohlenstoffatomen, vorzugsweise Methanol, Ethanol, n-Propanol, Isopropanol, tert.-Butanol oder Amylalkohol oder einem Gemisch daraus, und dem bei der Reaktion eingetragenen und/oder entstandenen Wasser. Ferner enthält die Lösung nicht verbrauchten Ammoniak sowie gegebenenfalls Spuren von nicht verbrauchtem Wasserstoffperoxid, von nicht umgesetztem Edukt (Keton) und von Nebenprodukten und Verunreinigungen des Eduktes. Dieses ist beispielsweise das dem jeweiligen Oxim analoge Imin. Wird als Keton beispielsweise technisch reines Cyclododecanon eingesetzt, sind die Nebenkomponenten und Verunreinigungen gering. In der Regel liegen sie deutlich unter 1 % bezogen auf das eingesetzte Keton.
Der Reaktoraustrag (1) wird zunächst in einem Mischer mit einer definierten Menge eines unpolaren Extraktionsmittels (2) versetzt, wobei darauf geachtet wird, dass die Menge an zugesetztem Extraktionsmittel eine ausreichende Löslichkeit für das Produkt (Oxim) bei der gewählten Extraktionstemperatur im Extraktor / Separator D aufweist.
Als Extraktionsmittel eignen sich sehr gut unpolare Kohlenwasserstoffe wie insbesondere aliphatische oder cycloaliphatische Kohlenwasserstoffe oder Gemische daraus, deren Siedepunkte oberhalb derjenigen vom verwendeten Alkohol und von Wasser liegen. Als nicht limitierende Beispiele seien Ethylcyclohexan, Dimethylcyclohexan, Isopropylcyclohexan (Hexahydrocumol), tert.-Butylcyclohexan, Cycloheptan, Cyclooctan, Cyclononan, Cyclodecan, Cycloundecan und Cyclododecan und alkylierte Derivate, vorzugsweise mit Alkylketten mit 1 bis 6 Kohlenstoffatomen davon genannt.

Die Mischung (3) wird beim erfindungsgemäßen Verfahren bei einer Temperatur von 60 - 90 °C bei Normaldruck oder einem vom Solvens gegebenen Überdruck gehalten. Bei dieser Temperatur liegt sie noch einphasig vor und es tritt keine Kristallisation des Oxims auf. Durch Variation der Parameter (Temperatur, Druck) und vor allem durch Zugabe von Kreiswasser (8) wird im Extraktor D das Reaktionsgemisch in die Zweiphasigkeit gebracht. Dabei trennt sich das Gemisch gemäß Bild 2 entlang der Konoden in zwei nicht miteinander mischbare Lösungen (4 und 7), ohne dass dabei Kristallisation von Oxim auftritt.

Das Oxim als Reaktionsprodukt und unter Umständen nicht umgesetztes Keton sowie gegebenenfalls weitere Nebenkomponenten gehen dabei vollständig oder nahezu vollständig in die lipophile Phase (4) über. Durch geeignete Extraktoren, beispielsweise durch einen Gegenstromextraktor, lässt sich dieser Trennschritt D (Zugabe Kreiswasser, Phasentrennung) verfahrenstechnisch optimieren. Hierbei können alle gängigen Extraktortypen verwendet werden wie zum Beispiel Mixer-Settler-Kaskaden, Zentrifugalextraktoren, und Kaskaden aus Zentrifugalextraktoren. Sie lassen sich sowohl im Kreuz- wie im Gegenstrom betreiben. Sollten nach dem Haupttrennschritt D noch Spuren von Oxim in der alkoholischen Phase 7 verbleiben, können diese durch eine nachgeschaltete Wäsche (nicht explizit eingezeichnet) mit frischem Extraktionsmittel aus Strom 7 herausgewaschsen werden. Das dabei verwendete Extraktionsmittel wird anschließend als Strom 2 im Trennschritt D weiter eingesetzt.

Restliche Spuren von Alkohol und Wasser werden aus dem Produktstrom (4) durch einen anschließenden Reinigungsschritt E, zum Beispiel eine kurze Destillationskolonne oder einen Stripper entfernt, wobei der abgetrennte Teilstrom 6, bestehend aus ammoniakalischen Alkohol-und Wasserresten, sowie einer kleinen Menge des unpolaren Kohlenwasserstoffs, in den Reaktor A oder vorteilhaft direkt in den Mischer C zurückgefahren wird. Die nachgeschaltete Reinigungsstufe E ist wichtig, damit die organische Phase (5) frei von Alkohol und Ammoniak gehalten wird, um bei der typischen Folgestufe der Oxime, der Beckmann-Umlagerung in konzentrierter Schwefelsäure, die Bildung von Dialkylsulfaten und Ammoniumsulfat zu unterdrücken.

Lösung 5 besteht aus dem Keton-Oxim in einem vorzugsweise aliphatischen oder cycloaliphatischen Kohlenwasserstoff. Zudem enthält sie Spuren von Edukt (Keton) und gegebenenfalls Nebenkomponenten der Ammoximation. Das Oxim lässt sich aus dieser Lösung direkt mit konzentrierter Schwefelsäure heraus extrahieren und zur Beckmann-Umlagerung einsetzen.

Die wässrig-alkoholische Phase (7) der Trennstufe D lässt sich durch eine nachgeschaltete, einoder mehrstufige Destillation leicht in ihre Bestandteile trennen. Die bevorzugten kurzkettigen Alkohole sieden in der Regel leichter als Wasser und werden als Azeotrop (9, 11) über Kopf abgezogen. Sie werden vereinigt (12), mit neuem Keton (13) versetzt (Apparat H) und in den Ammoximationsreaktor zurückgeführt (14), wo die Mischung mit Wasserstoffperoxid, Ammoniak und gegebenenfalls frischem Titansilikalit (zusammengefasst als Stoffstrom 15) versetzt wird.

Energetisch vorteilhaft erfolgt die Trennung von Strom 7 in Alkohol und Wasser in zwei oder mehreren Trennstufen (F und G). Im Sumpf (8) des ersten unscharfen Schnitts F verbleiben neben Wasser bis zu 20 Gew.-% Alkohol. Der Großteil dieser Mischung wird als Kreiswasserstrom in den Extraktor D zurückgeführt. Nur ein Teilstrom, der derjenigen Menge an Reaktionswasser entspricht, welches während der Ammoximation gebildet oder durch wässriges Wasserstoffperoxid und Ammoniak (15) in den Reaktor A eingetragen wurde, wird in einer nachgeschalteten Kolonne G vollständig vom Alkohol befreit und als Abwasser (10) ausgeschleust.

Die Aufarbeitungssequenzen D und E lassen sich optional auch in einer Extraktionskolonne zusammenfassen, wobei der eigentliche Extraktionsschritt D im Bereich des Zulaufs der Kolonne erfolgt. Im Sumpf der Extraktionskolonne kann direkt Strom 5 zur weiteren Umsetzung abgenommen werden, am Kopf wird Strom 7 abgenommen, der anschließend in den Destillationsstufen F und G weiter aufgetrennt wird. Ablagerungen von Oxim werden durch einen ausreichenden Rücklauf von kondensiertem Extraktionsmittel in der Extraktionskolonne vermieden.

Die Extraktion erfolgt vorzugsweise zwischen 0 und 130 °C und Drücken zwischen 0,1 und 10 bar.

Die im vorhergehenden für den Einsatz von mit Wasser gut oder vollständig mischbaren Alkoholen beschriebenen Verhältnisse gelten im Prinzip auch für andere mit Wasser gut oder vollständig mischbare unter den Reaktionsbedingungen inerte organische Flüssigkeiten. Alkohole werden jedoch bevorzugt.

Das erfindungsgemäße Prinzip der Aufarbeitung ist in Bild 2 für das ternäre System Ethanol, Wasser und Hexahydrocumol (in der Abbildung verkürzt als Hydrocumol bezeichnet) dargestellt. Eingezeichnet sind die Zusammensetzungen dieser drei Komponenten in den Stoffströmen 1, 3, 4 und 7. Die Zweiphasigkeit ist durch den gepunkteten Bereich skizziert.

Durch Zugabe von Hexahydrocumol im Mischer C bleibt das System zunächst noch in der Einphasigkeit (3). Durch Zugabe von Kreiswasser im Extraktor D wird das System in die Zweiphasigkeit gebracht (geregelt), wobei sich das System entlang der Konoden in die beiden nicht miteinander mischbaren Phasen 4 und 7 auftrennt, wobei die Grenzdestillationslinie überschritten wird. Die Reinigung in den nachgeschalteten Destillationkolonnen erfolgt dann entlang der Destillationslinien.

Die Angaben in Bild 2 beziehen sich auf einen konstanten Druck von 1,013 bar. Die Temperatur ist an jeder Stelle des Diagramms verschieden und entspricht der Siedetemperatur bei der jeweiligen Zusammensetzung und einem Gesamtdruck von 1,013 bar. Es sind Massenanteile angegeben.

## Patentansprüche

1. Verfahren zur Aufarbeitung eines Reaktionsgemisches, welches durch Ammoximation eines Ketons mit Wasserstoffperoxid und Ammoniak an einem titanhaltigen Katalysator in homogener Lösung gebildet wird,
**dadurch gekennzeichnet,**
**dass** es die Teilschritte
- Abtrennung des Katalysators aus der Reaktionsmischung,
- Abtrennung des Produktes,
- Abschleusung des Reaktionswassers und
- Rückführung des Lösemittels,
umfasst und dass die Abtrennung des gebildeten Keton-Oxims unter Verwendung mindestens einer flüssig-flüssig-Extraktion in einem ternären Lösemittelsystems erfolgt, wobei das ternäre Lösungsmittelsystem erstens aus einem oder mehreren vollständig oder gut mit Wasser mischbaren organischen Flüssigkeiten, zweitens aus Wasser und drittens aus einem mit Wasser und Alkohol nur partiell mischbaren organischen Lösemittel dessen Siedepunkt oberhalb der Siedepunkte von Alkohol und Wasser liegt, besteht.

2. Verfahren nach Anspruch 1,
**dadurch gekennzeichnet,**
**dass** als Lösemittel der Ammoximation eine polare, mit Wasser gut oder vollständig mischbare organische Flüssigkeit verwendet wird.

3. Verfahren nach einem der vorhergehenden Ansprüche,
**dadurch gekennzeichnet,**
**dass** als Lösemittel der Ammoximation ein kurzkettiger Alkohol, ausgewählt aus der Gruppe, die durch Methanol, Ethanol, n-Propanol, Isopropanol, tert.-Butanol, Amylalkohol oder ein Gemisch daraus, gebildet wird, verwendet wird.

4. Verfahren nach einem der vorhergehenden Ansprüche,
**dadurch gekennzeichnet,**
**dass** die flüssig-flüssig-Extraktion in einem ternären Lösemittelgemisch, bestehend aus Lösemittel, Wasser und Extraktionsmittel erfolgt unter Ausnutzung einer Mischungslücke.

5. Verfahren nach einem der vorhergehenden Ansprüche,
**dadurch gekennzeichnet,**
**dass** zur Extraktion eine unpolare, mit Wasser und Lösemittel nicht oder schlecht mischbare Flüssigkeit verwendet wird.

6. Verfahren nach einem der vorhergehenden Ansprüche,
**dadurch gekennzeichnet,**
**dass** als Extraktionsmittel ein unpolarer Kohlenwasserstoff verwendet wird, dessen Siedepunkt oberhalb derjenigen von Wasser und Lösemittel liegt.

7. Verfahren nach einem der vorhergehenden Ansprüche,
**dadurch gekennzeichnet,**
**dass** als Extraktionsmittel Ethylcyclohexan, Dimethylcyclohexan, Isopropylcyclohexan (Hexahydrocumol), tert.-Butylcyclohexan, Cycloheptan, Cyclooctan, Cyclononan, Cyclodecan, Cycloundecan oder Cyclododecan oder alkylierte Derivate davon verwendet werden.

8. Verfahren nach einem der vorhergehenden Ansprüche,
**dadurch gekennzeichnet,**
**dass** die flüssig-flüssig-Extraktion durch Zugabe eines Kreiswasserstroms geregelt wird.

9. Verfahren nach einem der vorhergehenden Ansprüche,
**dadurch gekennzeichnet,**
**dass** die flüssig-flüssig-Extraktion bei Temperaturen zwischen 0 und 130 °C und Drücken zwischen 0,1 und 10 bar erfolgt.

10. Verfahren nach einem der vorhergehenden Ansprüche,
**dadurch gekennzeichnet,**
**dass** das Extraktionsmittel in einem nachgeschalteten Reinigungsschritt von Resten hydrophiler Lösemittel befreit wird.

11. Verfahren nach Anspruch 3,
**dadurch gekennzeichnet,**
**dass** das bei der Ammoximation eingetragene Reaktionswasser aus der wässrigen, polaren organischen Phase abgetrennt wird und das polare organische Lösemittel in den Reaktor zurückgefahren wird.

12. Verfahren nach einem der vorhergehenden Ansprüche,
**dadurch gekennzeichnet,**
**dass** das Keton-Oxim aus der Extraktionslösung ohne weitere Reinigung in einer Beckmann-Umlagerung eingesetzt wird.

13. Verfahren nach einem der vorhergehenden Ansprüche,
**dadurch gekennzeichnet,**
**dass** das Keton ausgewählt wird aus der Gruppe Cyclooctanon, Cyclodecanon, Cyclododecanon, Cyclopentadecanon und Acetophenon.

## Claims

1. Process for working up a reaction mixture which is formed by ammoximation of a ketone by means of hydrogen peroxide and ammonia in homogeneous solution over a titanium-containing catalyst, **characterized in that** it comprises
- separating the catalyst from the reaction mixture,
- separating off the product,
- discharging the water of reaction and
- recirculating the solvent
and **in that** the ketone oxime formed is separated off using at least one liquid-liquid extraction in a ternary solvent system comprising firstly one or more organic liquids which are completely miscible or readily miscible with water, secondly water and thirdly and organic solvent which is only partly miscible with water and alcohol and whose boiling point is above the boiling points of alcohol and water.

2. Process according to Claim 1, **characterized in that** the solvent used in the ammoximation is a polar organic liquid which is readily miscible or completely miscible with water.

3. Process according to either of the preceding claims, **characterized in that** the solvent used in the ammoximation is a short-chain alcohol selected from the group consisting of methanol, ethanol, n-propanol, isopropanol, tert-butanol, amyl alcohol and mixtures thereof.

4. Process according to any of the preceding claims, **characterized in that** the liquid-liquid extraction is carried out in a ternary solvent mixture comprising solvent, water and extractant with exploitation of a miscibility gap.

5. Process according to any of the preceding claims, **characterized in that** a nonpolar liquid which is immiscible or sparingly miscible with water and the solvent is used for the extraction.

6. Process according to any of the preceding claims, **characterized in that** the extractant used is a nonpolar hydrocarbon whose boiling point is above those of water and the solvent.

7. Process according to any of the preceding claims, **characterized in that** the extractant used is ethylcyclohexane, dimethylcyclohexane, isopropylcyclohexane (hexahydrocumene), tert-butylcyclohexane, cycloheptane, cyclooctane, cyclononane, cyclodecane, cycloundecane or cyclododecane or an alkylated derivative thereof.

8. Process according to any of the preceding claims, **characterized in that** the liquid-liquid extraction is regulated by addition of a stream of circulated water.

9. Process according to any of the preceding claims, **characterized in that** the liquid-liquid extraction is carried out at temperatures of from 0 to 130°C and pressures of from 0.1 to 10 bar.

10. Process according to any of the preceding claims, **characterized in that** the extractant is freed of residual hydrophilic solvent in a downstream purification step.

11. Process according to Claim 3, **characterized in that** the water of reaction introduced in the ammoximation is separated from the aqueous, polar organic phase and the polar organic solvent is recirculated to the reactor.

12. Process according to any of the preceding claims, **characterized in that** the ketone oxime from the extraction solution is used in a Beckmann rearrangement without further purification.

13. Process according to any of the preceding claims, **characterized in that** the ketone is selected from the group consisting of cyclooctanone, cyclodecanone, cyclododecanone, cyclopentadecanone and acetophenone.

## Revendications

1. Procédé pour le traitement d'un mélange réactionnel qui est formé par ammoximation d'une cétone avec du peroxyde d'hydrogène et de l'ammoniaque sur un catalyseur contenant du titane en solution homogène, **caractérisé en ce qu'**il comprend les étapes partielles
- séparation du catalyseur du mélange réactionnel,
- séparation du produit,
- soutirage de l'eau de réaction et
- recyclage du solvant,
et **en ce que** la séparation de la cétone-oxime formée est réalisée en utilisant au moins une extraction liquide-liquide dans un système de solvants ternaire, le système de solvants ternaire étant constitué premièrement par un ou plusieurs liquides organiques complètement ou bien miscibles à l'eau, deuxièmement par de l'eau et troisièmement par un solvant organique qui n'est que partiellement miscible à l'eau et à l'alcool, dont le point d'ébullition est supérieur aux points d'ébullition de l'alcool et de l'eau.

2. Procédé selon la revendication 1, **caractérisé en ce qu'**on utilise comme solvant de l'ammoximation un liquide organique polaire, bien ou complètement miscible à l'eau.

3. Procédé selon l'une quelconque des revendications précédentes, **caractérisé en ce qu'**on utilise comme solvant de l'ammoximation un alcool à courte chaîne, choisi dans le groupe formé par le méthanol, l'éthanol, le n-propanol, l'isopropanol, le tert-butanol, l'alcool amylique ou un mélange de ceux-ci.

4. Procédé selon l'une quelconque des revendications précédentes, **caractérisé en ce que** l'extraction liquide-liquide est réalisée dans un mélange de solvants ternaire, constitué par un solvant, de l'eau et un agent d'extraction en exploitant une lacune de miscibilité.

5. Procédé selon l'une quelconque des revendications précédentes, **caractérisé en ce qu'**on utilise pour l'extraction un liquide non polaire, non ou peu miscible avec l'eau et le solvant.

6. Procédé selon l'une quelconque des revendications précédentes, **caractérisé en ce qu'**on utilise comme agent d'extraction un hydrocarbure non polaire dont le point d'ébullition est supérieur à celui de l'eau et du solvant.

7. Procédé selon l'une quelconque des revendications précédentes, **caractérisé en ce qu'**on utilise comme agent d'extraction de l'éthylcyclohexane, du diméthylcyclohexane, de l'isopropylcyclohexane (hexahydrocumène), du tert-butylcyclohexane, du cycloheptane, du cyclooctane, du cyclononane, du cyclodécane, du cyclo-undécane ou du cyclododécane ou des dérivés alkylés de ceux-ci.

8. Procédé selon l'une quelconque des revendications précédentes, **caractérisé en ce que** l'extraction liquide-liquide est réglée par addition d'un flux d'eau en circulation.

9. Procédé selon l'une quelconque des revendications précédentes, **caractérisé en ce que** l'extraction liquide-liquide est réalisée à des températures entre 0 et 130°C et des pressions entre 0,1 et 10 bars.

10. Procédé selon l'une quelconque des revendications précédentes, **caractérisé en ce que** l'agent d'extraction est libéré, dans une étape de purification ultérieure, de restes de solvant hydrophile.

11. Procédé selon la revendication 3, **caractérisé en ce que** l'eau de réaction introduite lors de l'ammoximation est séparée de la phase organique, polaire, aqueuse et le solvant organique polaire est recyclé dans le réacteur.

12. Procédé selon l'une quelconque des revendications précédentes, **caractérisé en ce que** la cétone-oxime de la solution d'extraction est utilisée sans autre purification dans une transposition de Beckmann.

13. Procédé selon l'une quelconque des revendications précédentes, **caractérisé en ce que** la cétone est choisie dans le groupe formé par la cyclooctanone, la cyclodécanone, la cyclododécanone, la cyclopentadécanone et l'acétophénone.
